(19)

Europäisches
Patentamt
European
Patent Office
Office européen
des brevets

(11) **EP 2 197 866 B1**

(12) **EUROPEAN PATENT SPECIFICATION**

(45) Date of publication and mention
of the grant of the patent:
**27.02.2013 Bulletin 2013/09**

(51) Int Cl.:
***C07D 307/46*** (2006.01)   ***C10L 1/02*** (2006.01)

(21) Application number: **08801980.7**

(22) Date of filing: **05.09.2008**

(86) International application number:
**PCT/EP2008/007413**

(87) International publication number:
**WO 2009/030507 (12.03.2009 Gazette 2009/11)**

(54) **HYDROXYMETHYLFURFURAL ETHERS FROM SUGARS AND HIGHER ALCOHOLS**

HYDROXYMETHYLFURFURALETHER AUS ZUCKERN UND HÖHEREN ALKOHOLEN

ETHERS HYDROXYMÉTHYLFURFURAUX DES SUCRES OU HMF ET ALCOOLS SUPÉRIEURS

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **07.09.2007 EP 07075775**

(43) Date of publication of application:
**23.06.2010 Bulletin 2010/25**

(73) Proprietor: **Furanix Technologies B.V**
**1014 BV Amsterdam (NL)**

(72) Inventors:
 • **GRUTER, Gerardus, Johannes, Maria**
  **NL-2106 BA Heemstede (NL)**
 • **MANZER, Leo, Ernest**
  **Wilmington, DE 19803 (US)**

(74) Representative: **Kortekaas, Marcel C.J.A.**
**Exter Polak & Charlouis B.V. (EP&C)**
**P.O. Box 3241**
**2280 GE Rijswijk (NL)**

(56) References cited:
 **EP-A- 1 834 950**    **WO-A-99/67409**
 **WO-A-2006/063220**   **WO-A-2007/104514**
 **DE-A1- 3 621 517**   **GB-A- 887 360**

 • **COTTIER L ET AL: "Photooxygenation of
  5-(hydroxymethyl)-2-furfural derivatives"
  BULLETIN DE LA SOCIETE CHIMIQUE DE
  FRANCE, SOCIETE FRANCAISE DE CHIMIE.
  PARIS, FR, 1986, pages 844-850, XP008086662
  ISSN: 0037-8968**
 • **BROWN DC W ET AL: "Dehydration reactions of
  fructose in nonaqueous media" JOURNAL OF
  CHEMICAL TECHNOLOGY AND
  BIOTECHNOLOGY, BLACKWELL SCIENTIFIC
  PUBLICATIONS. OXFORD, GB, vol. 32, no. 10,
  1982, pages 920-924, XP009095767 ISSN:
  0268-2575**
 • **OIKAWA M ET AL: "Parallel synthesis of tandem
  Ugi/Diels-Alder reaction products on a soluble
  polymer support directed toward split-pool
  realization of a small molecule library"
  TETRAHEDRON LETTERS, ELSEVIER,
  AMSTERDAM, vol. 46, no. 3, 17 January 2005
  (2005-01-17), pages 415-418, XP025384415 ISSN:
  0040-4039 [retrieved on 2005-01-17]**
 • **G. PAPANOV ET AL: "Synthesis of aromatic
  compounds, obtained at hydrolysis of extracted
  rose blossoms of rosa damascena Miller",
  TRAVAUX SCIENTIFIQUES - CHIMIE, vol. 28, no.
  5, 1990, pages 65-68,**

**Description**

Technical Field

The present invention concerns the use of an ether of 5-hydroxymethylfurfural (5-(hydroxymethyl)-2-furaldehyde, or HMF) and an alcohol with 6 or more carbon atoms as fuel or fuel additive.

Background Art

Fuel, fuel additives and various chemicals used in the petrochemical industry are derived from oil, gas and coal, all finite sources. Biomass, on the other hand, is considered a renewable source. Biomass is biological material (including biodegradable wastes) which can be used for the production of fuels or for industrial production of e.g. fibres, chemicals or heat. It excludes organic material which has been transformed by geological processes into substances such as coal or petroleum.

Production of biomass derived products for non-food applications is a growing industry. Bio-based fuels are an example of an application with strong growing interest..

Biomass contains sugars (hexoses and pentoses) that may be converted into value added products. Current biofuel activities from sugars are mainly directed towards the fermentation of sucrose or glucose into ethanol or via complete breakdown via Syngas to synthetic liquid fuels. EP 0641 854 describes the use of fuel compositions comprising of hydrocarbons and/or vegetable oil derivatives containing at least one glycerol ether to reduce particulate matter emissions.

More recently, the acid catalysed reaction of fructose has been re-visited, creating HMF as an intermediate of great interest. Most processes investigated have the disadvantage that HMF is not very stable at the reaction conditions required for its formation. Fast removal from the water-phase containing the sugar starting material and the acid catalyst has been viewed as a solution for this problem. Researchers at the University of Wisconsin-Madison have developed a process to make HMF from fructose. HMF can be converted into monomers for plastics, petroleum or fuel extenders, or even into fuel itself. The process by prof. James Dumesic and co-workers first dehydrates the fructose in an aqueous phase with the use of an acid catalyst (hydrochloric acid or an acidic ion-exchange resin). Salt is added to salt-out the HMF into the extracting phase. The extracting phase uses an inert organic solvent that favors extraction of HMF from the aqueous phase. The two-phase process operates at high fructose concentrations (10 to 50 wt %), achieves high yields (80% HMF selectivity at 90% fructose conversion), and delivers HMF in a separation-friendly solvent ( DUMESIC, James A, et al. "Phase modifiers promote efficient production of Hydroxymethylfurfural from fructose" . Science. 30 juni 2006, vol.312, no.5782, p.1933-1937). Although the HMF yields from this process are interesting, the multi-solvent process has cost-disadvantages due to the relatively complex plant design and because of the less than ideal yields when cheaper and less reactive hexoses than fructose, such as glucose or sucrose, are used as a starting material. HMF is a solid at room temperature which has to be converted in subsequent steps to useful products. Dumesic has reported an integrated hydrogenolysis process step to convert HMF into dimethylfuran (DMF), which is assumed to be an interesting gasoline additive.

In WO 2006/063220 a method is provided for converting fructose into 5-ethoxymethylfurfural (EMF) at 60 °C, using an acid catalyst either in batch during 24 hours or continuously via column elution during 17 hours. Applications of EMF were not discussed. The process is therefore very slow, and was found to be unsuitable for preparation of ethers with a higher alcohol, e.g., like in the preparation of 5-octylmethylfurfural, known from GB 887 360.

Also in copending patent application PCT/EP2007/002145 the manufacture of HMF ethers are described, including the use of such ethers as fuel or fuel additive. Indeed, both the methyl ether and the ethyl ether (methoxymethylfurfural, or MMF; ethoxyethylfurfural or EMF) were prepared and tested. The invention of the copending patent application, however, was limited to the use of primary aliphatic alcohols, and preferably primary C1-C5 alcohols. Higher alcohols, e.g. alcohols having 6 or more carbon atoms, preferably 8 or more carbon atoms, were not considered at all. Although MMF and EMF are useful as fuel or fuel additive, the inventors found that the ethers leave room for improvement, in particular when used in higher concentration blends with fuels such as gasoline, kerosene, diesel, biodiesel or green diesel. The inventors have therefore set out to overcome this shortfall.

Surprisingly, the inventors have found that ethers of HMF obtained from higher alcohols have superior blending properties compared to ethers obtained from methanol or ethanol analogues. The ethers of HMF with these alcohols may be produced in a reasonable yield from hexose containing feedstock or from HMF, with reduced levels of by-product formation and in a manner that does not require cumbersome process measures (such as 2-phase systems) or lengthy process times. Moreover, the inventors found that these ethers of HMF with higher alcohols may be best prepared in a process at higher temperatures and in the presence of additional solvents.

Disclosure of Invention

Accordingly, the present invention provides the use of an ether of 5-hydroxymethylfurfural and an alcohol having 6 or more carbon atoms as fuel or fuel additive. The invention further provides a fuel or fuel composition comprising an ether wherein the ether is an ether of 5-hydroxymethylfurfural and an alcohol having 6 or more carbon atoms.

Described is further a method for the manufacture of an ether of 5-hydroxymethylfurfural by reacting a hexose-containing starting material with a higher alcohol in the presence of an acid catalyst, performed in the presence of a solvent and at a temperature in the range of from 125 to 250 degrees Centigrade.

When the reaction product of the above method is used as such or when it is used as an intermediate for a subsequent conversion, the selectivity of the reaction is preferably high as the product is preferably pure. However, when the reaction product of the above method is used as a fuel, a fuel additive or as a fuel or a fuel additive intermediate, the reaction product does not necessarily need to be pure. Indeed, in the preparation of fuel and fuel additives from biomass, which in itself is a mixture of various monosaccharides, disaccharides and polysaccharides, the reaction product may contain non-interfering components such as levulinic acid derivatives and/or derivatives of pentoses and the like. For ease of reference, however, the method and the reaction product are described in terms of the reaction of a hexose-containing starting material, resulting in an ether of HMF.

The current invention also provides for the use of the reaction product made according to the above described method as fuel or as fuel additive. Fuels for blending with the product of the present invention include but are not limited to gasoline and gasoline-ethanol blends, kerosene, diesel, biodiesel (refers to a non-petroleum-based diesel fuel consisting of short chain alkyl (methyl or ethyl) esters, made by transesterification of vegetable oil, which can be used (alone, or blended with conventional petrodiesel), Fischer-Tropsch liquids (for example obtained from GTL, CTL or BTL gas-to-liquids/coal-to-liquids/biomass to liquids processes), diesel-biodiesel blends and green diesel and blends of diesel and/or biodiesel with green diesel (green diesel is a hydrocarbon obtained by hydrotreating biomass derived oils, fats, greases or pyrolysis oil; see for example the UOP report OPPORTUNITIES FOR BIORENEWABLES IN OIL REFINERIES FINAL TECHNICAL REPORT, SUBMITTED TO: U.S. DEPARTMENT OF ENERGY (DOE Award Number: DE-FG36-05GO15085). The product is a premium diesel fuel containing no sulfur and having a cetane number of 90 to 100). Fuels for blending with the product of the present invention may also include one or more other furanics, wherein the expression furanics is used to include all derivatives of furan and tetrahydrofuran. The invention also provides a fuel composition comprising a fuel element as described above and the reaction product made according to the described method.

Figures

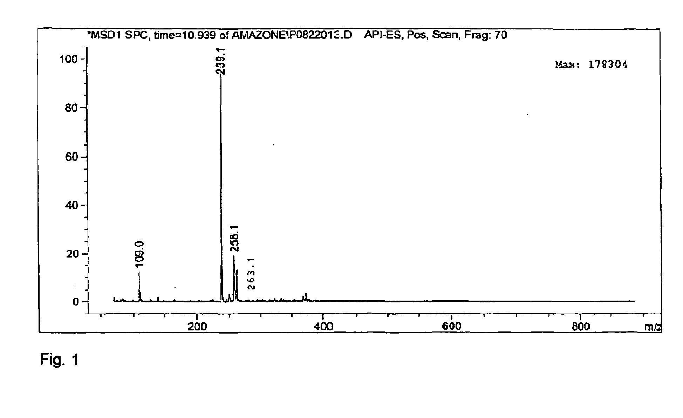

Figure 1 is the spectrum of 5-(hydroxymethyl)-furfural octyl ether, prepared by the described process, using a mass spectrometer in Chemical Ionization (C.I.) Mode.

Mode(s) for Carrying Out the Invention

Biomass resources are well known. The components of interest in biomass are the mono-, di- or polysaccharides (hereinafter referred to as hexose-containing starting material). Suitable 6-carbon monosaccharides include but are not limited to fructose, glucose, galactose, mannose and their oxidized, reduced, etherified, esterified and amidated derivatives, e.g. aldonic acid or alditol, with glucose being the most abundant, the most economic and therefore the most preferred monosaccharide albeit less reactive than fructose. On the other hand, the current inventors have also succeeded to convert sucrose, which is also available in great abundance. Other disaccharides that may be used include maltose, cellobiose and lactose. The polysaccharides that may be used include cellulose, inulin (a polyfructan), starch (a polyglucan) and hemi-cellulose. The polysaccharides and disaccharides are converted into their monosaccharide component (s) and dehydrated during the manufacture of the 5-HMF ether.

The higher alcohols used in the described method are typically monoalcohols, having a primary hydroxyl group. The alcohol commonly has an even number of carbon atoms, although synthetic higher alcohols may contain an odd number of carbon atoms as well. Higher alcohols may be saturated or unsaturated. Preferred are alcohols having 8 carbon atoms or more. Examples include: capryl alcohol (1-octanol); pelargonic alcohol (1-nonanol); capric alcohol (1-decanol); 1-dodecanol (lauryl alcohol); myristyl alcohol (1-tetradecanol); cetyl alcohol (1-hexadecanol); palmitoleyl alcohol (cis-9-hexadecan-1-ol); stearyl alcohol (1-octadecanol); isostearyl alcohol (16-methylheptadecan-1-ol); elaidyl alcohol (9E-octadecen-1-ol); oleyl alcohol (cis-9-octadecen-1-ol); linoleyl alcohol (9Z, 12Z-octadecadien-1-ol); elaidolinoleyl alcohol (9E, 12E-octadecadien-1-ol); linolenyl alcohol (9Z, 12Z, 15Z-octadecatrien-1-ol); elaidolinolenyl alcohol (9E, 12E, 15-E-octadecatrien-1-ol); and ricinoleyl alcohol (12-hydroxy-9-octadecen-1-ol); a diol. These alcohols are naturally occurring, allowing the synthesis of a fuel component or fuel additive that is fully derived from biomass. However, synthetic alcohols may be used as well, e.g., alcohols made by Fisher-Tropsch processes (a catalyzed chemical reaction in which carbon monoxide and hydrogen are converted into liquid hydrocarbons of various forms. Typical catalysts used are based on iron and cobalt. The principal purpose of this process is to produce a synthetic petroleum substitute, typically from coal or natural gas, for use as synthetic lubrication oil or as synthetic fuel, but the FT process is used for preparing alcohols as well). Another source of alcohols are the higher alcohols prepared via the Guerbet reaction (e.g., 2-ethylhexanol, prepared from butanol; "Selective synthesis of 2-ethyl-1-hexanol from n-butanol through the Guerbet reaction by using bifunctional catalysts based on copper or palladium precursors and sodium butoxide", by Carlo Carlini, Journal of Molecular Catalysis A: Chemical 212 (2004) 65-70).

Also blends of alcohols may be used, e.g., higher Guerbet alcohols made from a mixed alcohol feed or natural alcohols found as a blend in nature. The current method thus provides an excellent high value outlet for "contaminated" higher alcohols.

The amount of higher alcohol used during the manufacture of the HMF ether is preferably at least equimolar on the hexose content of the feedstock, but typically is used in much greater excess. Indeed, the alcohol (such as capryl alcohol) may be used as solvent or co-solvent. In such a case, a sufficient amount of alcohol is present to form the HMF ether.

The acid catalyst in the method described can be selected from amongst (halogenated) organic acids, inorganic acids, Lewis acids, ion exchange resins and zeolites or combinations and/or mixtures thereof. It may be a homogeneous catalyst, but heterogeneous catalysts are preferred for purification reasons. The HMF ethers can be produced with a protonic, Brønsted or, alternatively, a Lewis acid or with catalysts that have more than one of these acidic functionalities.

The protonic acid may be organic or inorganic. For instance, the organic acid can be selected from amongst oxalic acid, levulinic acid, maleic acid, trifluoro acetic acid (triflic acid), methansulphonic acid or para-toluenesulphonic acid. Alternatively, the inorganic acid can be selected from amongst (poly)phosphoric acid, sulphuric acid, hydrochloric acid, hydrobromic acid, nitric acid, hydroiodic acid, optionally generated in situ.

Certain salts may be used as catalyst, wherein the salt can be any one or more of $(NH_4)_2SO_4/SO_3$, ammonium phosphate, pyridinium chloride, triethylamine phosphate, pyridinium salts, pyridinium phosphate, pyridinium hydrochloride/hydrobromide/perbromate, DMAP, aluminium salts, Th and Zr ions, zirconium phosphate, Sc and lanthanide ions such as Sm and Y as their acetate or trifluoroactate (triflate) salt, Cr-, Al-, Ti-, Ca-, In-ions, $ZrOCl_2$, $VO(SO_4)_2$, $TiO_2$, V-porphyrine, Zr-, Cr-, Ti-porphyrine.

Lewis acids selected as dehydration catalyst can be any one of $ZnCl_2$, $AlCl_3$, $BF_3$.

Ion exchange resins can be suitable dehydration catalysts. Examples include Amberlite™ and Amberlyst™, Diaion™ and Levatit™. Other solid catalyst that may be used include natural clay minerals, zeolites, supported acids such as silica impregnated with mineral acids, heat treated charcoal, metal oxides, metal sulfides, metal salts and mixed oxides and mixtures thereof. The catalyst should be stable at the elevated reaction temperature, as defined hereafter.

An overview of catalysts that may be used in the method described may be found in Table 1 of the review article prepared by Mr. Lewkowski: "Synthesis, chemistry and applications of 5-hydroxymethylfurfural and its derivatives" Arkivoc. 2001, p.17-54.

The amount of catalyst may vary, depending on the selection of catalyst or catalyst mixture. For instance, the catalyst can be added to the reaction mixture in an amount varying from 0.01 to 40 mole % drawn on the hexose content of the biomass resource, preferably from 0.1 to 30 mole %, more preferably from 1 to 20 mole %.

In the preferred embodiment, the catalyst is a heterogeneous catalyst.

The temperature at which the reaction is performed may vary from 125 to 250 degrees Celsius, more preferably from 150 to 225 degrees Celsius. In general, temperatures higher than 300 are less preferred as the selectivity of the reaction reduces and as many byproducts occur, inter alia caramelisation of the sugar. Performing the reaction below the lowest temperature is also less preferable because of the low reaction rate. As the reactions are carried out above the boiling temperature of water, the reactions are preferably carried out under pressure, e.g., 10 bar nitrogen or higher.

The hexose-containing starting material is typically dissolved or suspended in a solvent which can (to some extent) be the alcohol reactant, in order to facilitate the reaction. The solvent may be selected form the group consisting of water, sulfoxides, preferably DMSO, ketones, preferably methyl ethylketone, methylisobutylketone and acetone or mixtures of two or more of the above solvents. Also so-called ionic liquids may be used. The latter refers to a class of inert ionic compounds with a low melting point, which may therefore be used as solvent. Examples thereof include e.g., 1-H-3-methyl imidazolium chloride, discussed in "Dehydration of fructose and sucrose into 5-hydroxymethylfurfural in the presence of 1-H-3-methyl imidazolium chloride acting both as solvent and catalyst", by Claude Moreau et al, Journal of Molecular Catalysis A: Chemical 253 (2006) 165-169.

Basically a sufficient amount of solvent is preferably present to dissolve or to suspend the starting material and to limit undesired side-reactions.

The method described may be carried out in a batch process or in a continuous process, with or without recycle of (part of) the product stream to control the reaction temperature (recycle via a heat exchanger). For instance, the method can be performed in a continuous flow process. In such method, homogenous catalysts may be used and the residence time of the reactants in the flow process is between 0.1 second and 10 hours, preferably from 1 second to 1 hours, more preferably from 5 seconds to 20 minutes.

Alternatively, the continuous flow process may be a fixed bed continuous flow process or a reactive (catalytic) distillation process with a heterogeneous acid catalyst. To initiate or regenerate the heterogeneous acid catalyst or to improve performance, an inorganic or organic acid may be added to the feed of the fixed bed or reactive distillation continuous flow process. In a fixed bed process, the liquid hourly space velocity (LHSV) can be from 1 to 1000, preferably from 5 to 500, more preferably from 10 to 250 and most preferably from 25 to 100 $min^{-1}$.

The above process results in a stable HMF ether, which can then be used as such or be converted into a further derivative before being used as fuel and/or as fuel additive. The inventors are of the opinion that some of the products prepared by the method described herein. Thus, the ethers made with C6 to C20 alcohols, preferably C8 to C14 alcohols are new and are excellent fuel components or fuel additives. Since these alcohols may be made from biomass, this might open a class of products that are fully biomass-derived. Accordingly, these new ethers are claimed as well.

The HMF ethers of the invention can also be used as or can be converted to compounds that can be used as solvent, as a detergent, as a surfactant, as monomer in a polymerization (such as 2,5-furan dicarboxylic acid or FDCA), as fine chemical or pharmaceutical intermediate, or in other applications. Oxidation of the HMF ethers using an appropriate catalyst under appropriate conditions such as for example described for p-xylene with a NHPI/Co(OAc)$_2$/MnOAc)$_2$ catalyst system in Adv. Synth. Catal. 2001, 343, 220-225 or such as described for HMF with a Pt/C catalyst system at pH < 8 in EP 0 356 703 or or such as described for HMF with a Pt/C catalyst system at pH > 7 in FR 2 669 634, all with air as an oxidant, resulted in the formation of 2,5-Furan dicarboxylic acid (FDCA).

The invention concerns the use of the HMF ethers prepared by this method as fuel and/or as fuel additive. Of particular interest is the use of the ethers in diesel, biodiesel or "green diesel", given its (much) greater solubility therein than ethanol. Conventional additives and blending agents for diesel fuel may be present in the fuel compositions of this invention in addition to the above mentioned fuel components. For example, the fuels of this invention may contain conventional quantities of conventional additives such as cetane improvers, friction modifiers, detergents, antioxidants and heat stabilizers, for example. Especially preferred diesel fuel formulations of the invention comprise diesel fuel hydrocarbons and HMF ether as above described together with peroxidic or nitrate cetane improvers such as ditertiary butyl peroxide, amyl nitrate and ethyl hexyl nitrate for example.

Examples are enclosed to illustrate the method described and the suitability of the products prepared therefrom as fuel. The examples are not meant to limit the scope of the invention.

**Comparative Example 1.**

In the manner described in WO2006063220, however using n-octanol instead of ethanol it was tried to prepare 5-(oxtyloxymethyl)furfural from fructose in batch mode. The stirred mixture could not be heated to reflux, as this inactivated the catalyst. On the other hand, when performing the reaction at the boiling temperature of ethanol (about 80 degrees Centigrade) no product could be isolated even after 24 hours. Moreover, performing the experiment at reflux temperatures (above the boiling point of water) caused solubility issues, most likely due to the effective removal of water from the reaction mixture.

**Example 1. (Reference)**

In a 7.5 ml batch reactor, 0.053 mmol fructose in octanol/water 90/10 v/v, was reacted for 1 hour at a temperature of 150 degrees Celsius with 9 mg acid catalyst. Two main furan peaks were observed in the UV spectrum. Mass spectrometry identified these products as HMF and 5-(octyloxymethyl)furfural (OMF). Selectivities and conversions for catalysts used in this example can be found in table below.

Conversion of substrate, selectivity and yield of furan derivatives were calculated according to the following formulae:

$$X = 100 * m_{r\ substrate} / m_{0\ substrate}$$

| | |
|---|---|
| X | conversion (%) |
| $m_r$ substrate | amount of reacted substrate (mg) |
| $m_0$ substrate | amount of substrate in feed (mg) |

$$S_{compound} = 100 * n_{r\ substrate} / n_{0\ substrate}$$

| | |
|---|---|
| $S_{compound}$ | selectivity to compound (%) |
| $n_r$ substrate | moles of substrate reacted |
| $n_0$ substrate | moles of substrate in feed |

$$Yield = 100 * n_{product} / n_{0\ substrate}$$

| | |
|---|---|
| Yield | yield (%) |
| $n_{product}$ | moles of product formed |

Table 1. Conversion and selectivities for the dehydration of fructose in the presence of 1-octanol.

| Catalyst | Conversion | selectivity. HMF (%) | selectivity. OMF (%) | selectivity. Lev Acid (%) |
|---|---|---|---|---|
| CrCl2 | 83.7 | 1.2 | 11.4 | 0.8 |
| Sm(III)Triflate | 88.4 | 0 | 7.3 | 12.6 |
| Amberlyst36 | 100 | 0.1 | 16.4 | 14 |

Example 2. (Reference)

In a typical experiment, similar to Example 1, 65 mg of glucose (Glc) or fructose (Frc) as substrate and 0.8 ml of n-octanol were added in a reactor coated inside with Teflon. No water was added. The mixture reacted under nitrogen (12.5 bar) in the presence of a solid acid catalyst (6.5 mg) for 3 h at 135°C. The two main peaks observed in the UV spectrum were identified as HMF and 5-(octyloxymethyl)fufural (OMF). The results are listed in Table 2. This example illustrates that the reaction can be carried out (preferred embodiment) without added water. In this experiment, the selectivity was calculated slightly different, based on the formula:

$$Selectivity = 100 * n_t (product) / [n_0 (substrate) - n_t (substrate)]$$

Where:
$n_0$- the initial number of moles
$n_t$- the number the moles of a compound at time "t".

| Substrate | Catalyst | Conversion (%) | HMF Selectivity (%) | OMF selectivity (%) |
|---|---|---|---|---|
| Glc | $CrCl_2$ | 100 | 0 | 4 |
| Frc | $CrCl_2$ | 100 | 0 | 12 |
| Frc | Montmorillonite K 5 | 100 | 0 | 2 |

**Analytical Method**

The reaction products were quantified with the aid of HPLC-analysis with an internal standard (saccharine, Sigma Aldrich). An Agilent 1100 series chromatograph, equipped with UV and ELSD detectors, was used. Stationary phase was reverse phase C18 (Sunfire 3.5 $\mu$m, 4.6x100mm, Waters) column. A gradient elution at a constant flow 0.6 ml/min and temperature 40°C was used according to the following scheme.

| Time | H2O(vol%) | MeOH (vol%) | MeCN (vol%) | Flow (ml/min) | T(C) |
|---|---|---|---|---|---|
| Initial | 95 | 0 | 5 | 1 | 40 |

(continued)

| Time | H2O(vol%) | MeOH (vol%) | MeCN (vol%) | Flow (ml/min) | T(C) |
|---|---|---|---|---|---|
| 1 | 89 | 3 | 8 | 1 | 40 |
| 8 | 25 | 3 | 72 | 1 | 40 |

The product was characterized with LC-MS (CI) (See Figure 1). Molecular mass of OMF is 238.3 g/mol.

## Example 3. Diesel fuel application

Fuel solubility

Fuel solubility is a primary concern for diesel fuel applications. Not all highly polar oxygenates have good solubility in the current commercial diesel fuels. Results show that in the 5 vol%, in the 25 vol% and in the 40 vol% blends of OMF with commercial diesel, both liquid blend components are completely miscible. In a comparative set of experiments it was shown that ethoxymethylfurfural (EMF) is completely miscible in a 5 vol% blend with commercial diesel, but that phase separation occurs with the 25 vol% and with the 40 vol% blends of EMF and diesel.

References

- DUMESIC, James A, et al. "Phase modifiers promote efficient production of Hydroxymethylfurfural from fructose" . Science. 30 June 2006, vol.312, no.5782, p.1933-1937.
- WO 2006/063220
- Chapter 15 of Advanced Organic Chemistry, by Jerry March, and in particular under reaction 5-4. (3rd ed., © 1985 by John Wiley & Sons, pp. 684-685).
- LEWKOWSKI, Jaroslaw. Synthesis, chemistry and applications of 5-hydroxymethylfurfural and its derivatives. Arkivoc. 2001, p.17-54.
- MOREAU, Claude, et al. "Dehydration of fructose and sucrose into 5-hydroxymethylfurfural in the presence of 1-H-3-methyl imidazolium chloride acting both as solvent and catalyst", Journal of Molecular Catalysis A: Chemical 253 (2006) p. 165-169.
- EP 0641 854
- UOP report OPPORTUNITIES FOR BIORENEWABLES IN OIL REFINERIES FINAL TECHNICAL REPORT, SUBMITTED TO: U.S. DEPARTMENT OF ENERGY (DOE Award Number: DE-FG36-05GO15085))
- Adv. Synth. Catal. 2001, 343, 220-225
- EP 0 356 703
- FR 2 669 634

## Claims

1.    Use of an ether of 5-hydroxymethylfurfural and an alcohol having 6 or more carbon atoms as fuel or fuel additive.

2.    Use according to claim 1, wherein the ether is an ether of 5-hydroxymethylfurfural and an alcohol having 6 or more carbon atoms, with the exception of 5-(hydroxymethyl)furfural octyl ether.

3.    Use according to claim 1, wherein the ether is 5-(hydroxymethyl)furfural decyl ether; or
5-(hydroxymethyl)furfural dodecyl ether; or
5-(hydroxymethyl)furfural octadecyl ether; or
5-(hydroxymethyl)furfural palmitoleyl ether; or
5-(hydroxymethyl)furfural cis-9-hexadecan-1-yl ether; or
5-(hydroxymethyl)furfural isostearyl ether; or
5-(hydroxymethyl)furfural elaidyl ether; or
5-(hydroxymethyl)furfural oleyl ether; or
5-(hydroxymethyl)furfural linoleyl ether; or
5-(hydroxymethyl)furfural elaidolinoleyl ether; or

5-(hydroxymethyl)furfural linolenyl ether; or
5-(hydroxymethyl)furfural elaidolinolenyl ether; or
5-(hydroxymethyl)furfural ricinoleyl ether; or
mixed ethers of 5-(hydroxymethyl)furfural with FT alcohols having 7 to 20 carbon atoms; or mixed ethers of 5-(hydroxymethyl)furfural and Guerbet alcohols having 8 to 20 carbon atoms.

4. A fuel or fuel composition comprising an ether, wherein the ether is an ether of 5-hydroxymethylfurfural and an alcohol having 6 or more carbon atoms, which is blended with one or more of gasoline and gasoline-ethanol blends, kerosene, diesel, biodiesel (a non-petroleum-based diesel fuel consisting of short chain alkyl (methyl or ethyl) esters, made by transesterification of vegetable oil), Fischer-Tropsch liquids, diesel-biodiesel blends and green diesel (a hydrocarbon obtained by hydrotreating biomass derived oils, fats, greases or pyrolysis oil; containing no sulfur and having a cetane number of 90 to 100) and blends of diesel and/or biodiesel with green diesel and other derivatives of furan or tetrahydrofuran.

5. Fuel or fuel composition according to claim 4, wherein the ether is an ether of 5-hydroxymethylfurfural and an alcohol having 6 or more carbon atoms, with the exception of 5-(hydroxymethyl)furfural octyl ether.

6. Fuel or fuel composition according to claim 4, wherein the ether is 5-(hydroxymethyl)furfural dodecyl ether; or
5-(hydroxymethyl)furfural octadecyl ether; or
5-(hydroxymethyl)furfural palmitoleyl ether; or
5-(hydroxymethyl)furfural cis-9-hexadecan-1-yl ether; or
5-(hydroxymethyl)furfural isostearyl ether; or
5-(hydroxymethyl)furfural elaidyl ether; or
5-(hydroxymethyl)furfural oleyl ether; or
5-(hydroxymethyl)furfural linoleyl ether; or
5-(hydroxymethyl)furfural elaidolinoleyl ether; or
5-(hydroxymethyl)furfural linolenyl ether; or
5-(hydroxymethyl)furfural elaidolinolenyl ether; or
5-(hydroxymethyl)furfural ricinoleyl ether; or
mixed ethers of 5-(hydroxymethyl)furfural with FT alcohols having 7 to 20 carbon atoms; or mixed ethers of 5-(hydroxymethyl)furfural and Guerbet alcohols having 8 to 20 carbon atoms.

7. Ether of 5-hydroxymethylfurfural and an alcohol having 6 or more carbon atoms selected from
5-(hydroxymethyl)furfural dodecyl ether; or
5-(hydroxymethyl)furfural octadecyl ether; or
5-(hydroxymethyl)furfural palmitoleyl ether; or
5-(hydroxymethyl)furfural cis-9-hexadecan-1-yl ether; or
5-(hydroxymethyl)furfural isostearyl ether; or
5-(hydroxymethyl)furfural elaidyl ether; or
5-(hydroxymethyl)furfural oleyl ether; or
5-(hydroxymethyl)furfural linoleyl ether; or
5-(hydroxymethyl)furfural elaidolinoleyl ether; or
5-(hydroxymethyl)furfural linolenyl ether; or
5-(hydroxymethyl)furfural elaidolinolenyl ether; or
5-(hydroxymethyl)furfural ricinoleyl ether; or
mixed ethers of 5-(hydroxymethyl)furfural with FT alcohols having 7 to 20 carbon atoms; or mixed ethers of 5-(hydroxymethyl)furfural and Guerbet alcohols having 8 to 20 carbon atoms.

**Patentansprüche**

1. Verwendung eines Ethers aus 5-(Hydroxymethyl)furfural und einem Alkohol mit 6 oder mehr Kohlenstoffatomen als Kraftstoff oder Kraftstoffadditiv.

2. Verwendung nach Anspruch 1, wobei der Ether ein Ether aus 5-(Hydroxymethyl)furfural und einem Alkohol mit 6 oder mehr Kohlenstoffatomen mit Ausnahme von 5-(Hydroxymethyl)furfuraloctylether ist.

3. Verwendung nach Anspruch 1, wobei der Ether 5-(Hydroxymethyl)furfuraldecylether oder

5-(Hydroxymethyl)furfuraldodecylether oder
5-(Hydroxymethyl)furfaloctadecylether oder
5-(Hydroxymethyl)furfalpalmitoleylether oder
5-(Hydroxymethyl)furfural-cis-9-hexadecan-1-ylether oder
5-(Hydroxymethyl)furfalisostearylether oder
5-(Hydroxymethyl)furfalelaidylether oder
5-(Hydroxymethyl)furfaloleylether oder
5-(Hydroxymethyl)furfallinoleylether oder
5-(Hydroxymethyl)furfalelaidolinoleylether oder
5-(Hydroxymethyl)furfallinolenylether oder
5-(Hydroxymethyl)furfalelaidolinolenylether oder
5-(Hydroxymethyl)furfalricinoleylether oder
ein gemischter Ether aus 5-(Hydroxymethyl)furfural mit FT-Alkoholen mit 7 bis 20 Kohlenstoffatomen oder ein gemischter Ether aus 5-(Hydroxymethyl)furfural und Guerbet-Alkoholen mit 8 bis 20 Kohlenstoffatomen ist.

4. Kraftstoff oder Kraftstoffzusammensetzung, umfassend einen Ether, wobei der Ether ein Ether aus 5-(Hydroxymethyl)furfural und einem Alkohol mit 6 oder mehr Kohlenstoffatomen ist, der mit einer oder mehreren der Substanzen aus Benzin und Benzin-Ethanolgemischen, Kerosin, Diesel, Biodiesel (ein Dieselkraftstoff nicht auf Erdölbasis, der aus kurzkettigen Alkyl- (Methyl- oder Ethyl-) estern besteht, die durch Umesterung von pflanzlichem Öl hergestellt sind), Fischer-Tropsch-Flüssigkeiten, Diesel-Biodieselgemischen und grünem Diesel (ein Kohlenwasserstoff, der durch Hydrotreating von aus Biomasse stammenden Ölen, Fetten, Schmierölen oder Pyrolyseölen erhalten wird, kein Schwefel enthält und eine Cetanzahl von 90 bis 100 aufweist) und Mischungen aus Diesel und/oder Biodiesel mit grünem Diesel und anderen Furan- oder Tetrahydrofuranderivaten gemischt wird.

5. Kraftstoff oder Kraftstoffzusammensetzung nach Anspruch 4, wobei der Ether ein Ether aus 5-(Hydroxymethyl) furfural und einem Alkohol mit 6 oder mehr Kohlenstoffatomen mit Ausnahme von 5-(Hydroxymethyl)furfuraloctylether ist.

6. Kraftstoff oder Kraftstoffzusammensetzung nach Anspruch 4, wobei der Ether
5-(Hydroxymethyl)furfuraldodecylether oder
5-(Hydroxymethyl)furfaloctadecylether oder
5-(Hydroxymethyl)furfalpalmitoleylether oder
5-(Hydroxymethyl)furfural-cis-9-hexadecan-1-ylether oder
5-(Hydroxymethyl)furfalisostearylether oder
5-(Hydroxymethyl)furfalelaidylether oder
5-(Hydroxymethyl)furfaloleylether oder
5-(Hydroxymethyl)furfallinoleylether oder
5-(Hydroxymethyl)furfalelaidolinoleylether oder
5-(Hydroxymethyl)furfallinolenylether oder
5-(Hydroxymethyl)furfalelaidolinolenylether oder
5-(Hydroxymethyl)furfalricinoleylether oder
ein gemischter Ether aus 5-(Hydroxymethyl)furfural mit FT-Alkoholen mit 7 bis 20 Kohlenstoffatomen oder ein gemischter Ether aus 5-(Hydroxymethyl)furfural und Guerbet-Alkoholen mit 8 bis 20 Kohlenstoffatomen ist.

7. Ether aus 5-(Hydroxymethyl)furfural und einem Alkohol mit 6 oder mehr Kohlenstoffatomen, der aus
5-(Hydroxymethyl)furfuraldodecylether oder
5-(Hydroxymethyl)furfaloctadecylether oder
5-(Hydroxymethyl)furfalpalmitoleylether oder
5-(Hydroxymethyl)furfural-cis-9-hexadecan-1-ylether oder
5-(Hydroxymethyl)furfalisostearylether oder
5-(Hydroxymethyl)furfalelaidylether oder
5-(Hydroxymethyl)furfaloleylether oder
5-(Hydroxymethyl)furfallinoleylether oder
5-(Hydroxymethyl)furfalelaidolinoleylether oder
5-(Hydroxymethyl)furfallinolenylether oder
5-(Hydroxymethyl)furfalelaidolinolenylether oder
5-(Hydroxymethyl)furfalricinoleylether oder
gemischten Ethern aus 5-(Hydroxymethyl)furfural mit FT-Alkoholen mit 7 bis 20 Kohlenstoffatomen oder gemischten Ethern aus 5-(Hydroxymethyl)furfural und Guerbet-Alkoholen mit 8 bis 20 Kohlenstoffatomen ausgewählt ist.

**Revendications**

1. Utilisation d'un éther de 5-hydroxyméthylfurfural et d'un alcool comportant 6 atomes de carbone ou plus en tant que carburant ou additif de carburant.

2. Utilisation selon la revendication 1, dans laquelle l'éther est un éther de 5-hydroxyméthylfurfural et d'un alcool comportant 6 atomes de carbone ou plus, à l'exception de l'éther 5-(hydroxyméthyl)furfural-octylique.

3. Utilisation selon la revendication 1, dans laquelle l'éther est l'éther 5-(hydroxyméthyl)furfural-décylique ; ou
l'éther 5-(hydroxyméthyl)furfural-dodécylique ; ou
l'éther 5-(hydroxyméthyl)furfural-octadécylique ; ou
l'éther 5-(hydroxyméthyl)furfural-palmitoléylique ; ou
l'éther 5-(hydroxyméthyl)furfural-cis-9-hexadécan-1-ylique ; ou
l'éther 5-(hydroxyméthyl)furfural-isostéarylique ; ou
l'éther 5-(hydroxyméthyl)furfural-élaïdylique ; ou
l'éther 5-(hydroxyméthyl)furfural-oléylique ; ou
l'éther 5-(hydroxyméthyl)furfural-linoléylique ; ou
l'éther 5-(hydroxyméthyl)furfural-élaïdolinoléylique ; ou
l'éther 5-(hydroxyméthyl)furfural-linolénylique ; ou
l'éther 5-(hydroxyméthyl)furfural-élaïdolinolénylique ; ou
l'éther 5-(hydroxyméthyl)furfural-ricinoléylique ; ou
les éthers mixtes de 5-(hydroxyméthyl)furfural et d'alcools FT comportant de 7 à 20 atomes de carbone ; ou
les éthers mixtes de 5-(hydroxyméthyl)furfural et d'alcools de Guerbet comportant de 8 à 20 atomes de carbone.

4. Carburant ou composition de carburant comprenant un éther, dans lequel l'éther est un éther de 5-hydroxyméthylfurfural et d'un alcool comportant 6 atomes de carbone ou plus, qui est mélangé à un ou plusieurs parmi l'essence et les mélanges d'essence-éthanol, le kérosène, le diesel, le biodiesel (un carburant diesel non dérivé du pétrole constitué d'esters d'alkyle (méthyle ou éthyle) à chaîne courte, fabriqués par transestérification d'huile végétale), les liquides de Fischer-Tropsch, les mélanges de diesel-biodiesel et le diesel vert (un hydrocarbure obtenu par hydrotraitement d'huiles, de matières grasses, de graisses ou d'huile de pyrolyse dérivées de la biomasse ; ne contenant pas de soufre et ayant un indice de cétane de 90 à 100) et les mélanges de diesel et/ou de biodiesel avec du diesel vert et d'autres dérivés de furanne ou de tétrahydrofuranne.

5. Carburant ou composition de carburant selon la revendication 4, dans lequel l'éther est un éther de 5-hydroxyméthylfurfural et d'un alcool comportant 6 atomes de carbone ou plus, à l'exception de l'éther 5-(hydroxyméthyl)furfural-octylique.

6. Carburant ou composition de carburant selon la revendication 4, dans lequel l'éther est l'éther 5-(hydroxyméthyl) furfural-dodécylique ; ou
l'éther 5-(hydroxyméthyl)furfural-octadécylique ; ou
l'éther 5-(hydroxyméthyl)furfural-palmitoléylique ; ou
l'éther 5-(hydroxyméthyl)furfural-cis-9-hexadécan-1-ylique ; ou
l'éther 5-(hydroxyméthyl)furfural-isostéarylique ; ou
l'éther 5-(hydroxyméthyl)furfural-élaïdylique ; ou
l'éther 5-(hydroxyméthyl)furfural-oléylique ; ou
l'éther 5-(hydroxyméthyl)furfural-linoléylique ; ou
l'éther 5-(hydroxyméthyl)furfural-élaïdolinoléylique ; ou
l'éther 5-(hydroxyméthyl)furfural-linolénylique ; ou
l'éther 5-(hydroxyméthyl)furfural-élaïdolinolénylique ; ou
l'éther 5-(hydroxyméthyl)furfural-ricinoléylique ; ou
les éthers mixtes de 5-(hydroxyméthyl)furfural et d'alcools FT comportant de 7 à 20 atomes de carbone ; ou
les éthers mixtes de 5-(hydroxyméthyl)furfural et d'alcools de Guerbet comportant de 8 à 20 atomes de carbone.

7. Éther de 5-hydroxyméthylfurfural et d'un alcool comportant 6 atomes de carbone ou plus choisi parmi
l'éther 5-(hydroxyméthyl)furfural-dodécylique ; ou

l'éther 5-(hydroxyméthyl)furfural-octadécylique ; ou
l'éther 5-(hydroxyméthyl)furfural-palmitoléylique ; ou
l'éther 5-(hydroxyméthyl)furfural-cis-9-hexadécan-1-ylique ; ou
l'éther 5-(hydroxyméthyl)furfural-isostéarylique ; ou
l'éther 5-(hydroxyméthyl)furfural-élaïdylique ; ou
l'éther 5-(hydroxyméthyl)furfural-oléylique ; ou
l'éther 5-(hydroxyméthyl)furfural-linoléylique ; ou
l'éther 5-(hydroxyméthyl)furfural-élaïdolinoléylique ; ou
l'éther 5-(hydroxyméthyl)furfural-linolénylique ; ou
l'éther 5-(hydroxyméthyl)furfural-élaïdolinolénylique ; ou
l'éther 5-(hydroxyméthyl)furfural-ricinoléylique ; ou
les éthers mixtes de 5-(hydroxyméthyl)furfural et d'alcools FT comportant de 7 à 20 atomes de carbone ; ou
les éthers mixtes de 5-(hydroxyméthyl)furfural et d'alcools de Guerbet comportant de 8 à 20 atomes de carbone.

Fig. 1

## REFERENCES CITED IN THE DESCRIPTION

### Patent documents cited in the description

- EP 0641854 A
- WO 2006063220 A
- GB 887360 A
- EP 2007002145 W
- EP 0356703 A
- FR 2669634

### Non-patent literature cited in the description

- **DUMESIC, JAMES A et al.** Phase modifiers promote efficient production of Hydroxymethylfurfural from fructose. *Science,* 30 June 2006, vol. 312 (5782), 1933-1937
- **CARLO CARLINI.** Selective synthesis of 2-ethyl-1-hexanol from n-butanol through the Guerbet reaction by using bifunctional catalysts based on copper or palladium precursors and sodium butoxide. *Journal of Molecular Catalysis A: Chemical,* 2004, vol. 212, 65-70
- **MR. LEWKOWSKI.** Synthesis, chemistry and applications of 5-hydroxymethylfurfural and its derivatives. *Arkivoc.,* 2001, 17-54
- **CLAUDE MOREAU et al.** Dehydration of fructose and sucrose into 5-hydroxymethylfurfural in the presence of 1-H-3-methyl imidazolium chloride acting both as solvent and catalyst. *Journal of Molecular Catalysis A: Chemical,* 2006, vol. 253, 165-169
- *Adv. Synth. Catal.,* 2001, vol. 343, 220-225
- **JERRY MARCH.** Advanced Organic Chemistry. John Wiley & Sons, 1985, 684-685
- **LEWKOWSKI, JAROSLAW.** Synthesis, chemistry and applications of 5-hydroxymethylfurfural and its derivatives. *Arkivoc.,* 2001, 17-54
- **MOREAU, CLAUDE et al.** Dehydration of fructose and sucrose into 5-hydroxymethylfurfural in the presence of 1-H-3-methyl imidazolium chloride acting both as solvent and catalyst. *Journal of Molecular Catalysis A: Chemical,* 2006, vol. 253, 165-169